(19) Europäisches Patentamt

European Patent Office

Office européen des brevets

(11) **EP 1 249 203 A2**

(12) **EUROPEAN PATENT APPLICATION**

(43) Date of publication:
**16.10.2002 Bulletin 2002/42**

(51) Int Cl.[7]: **A61B 5/0285**, A61B 5/022

(21) Application number: **01130118.1**

(22) Date of filing: **18.12.2001**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **09.04.2001 JP 2001109783**

(71) Applicant: **Colin Corporation
Komaki-shi, Aichi-ken (JP)**

(72) Inventor: **Ogura, Toshihiko
Komaki-shi, Aichi-ken (JP)**

(74) Representative:
**Winter, Brandl, Fürniss, Hübner, Röss, Kaiser,
Polte Partnerschaft
Patent- und Rechtsanwaltskanzlei
Alois-Steinecker-Strasse 22
85354 Freising (DE)**

(54) **Pulse-wave-propagation-velocity measuring apparatus**

(57)    An apparatus (10) for measuring a velocity of propagation of a pulse wave between two portions of a living subject, based on a distance, and a time, of propagation of the pulse wave between the two portions, the apparatus comprising a propagation-distance determining means (90) for determining the distance of propagation of the pulse wave, based on stature-related information that is related to a stature of the subject and age-related information that is related to an age of the subject, according to a predetermined relationship between (A) propagation distance and (B) (b1) stature-related information and (b) age-related information; and a propagation-velocity determining means (94) for determining the velocity of propagation of the pulse wave based on at least the distance of propagation determined by the propagation-distance determining means.

FIG. 1

**Description**

BACKGROUND OF THE INVENTION

Field of the Invention

The present invention relates to an apparatus for measuring a velocity at which a pulse wave propagates in a living subject.

Related Art Statement

Pulse-wave propagation velocity PWV (m/sec) is used to make various sorts of diagnoses. For example, since pulse-wave propagation velocity PWV increases as hardness of arteries increases, the velocity PWV is used to diagnose arteriosclerosis. In addition, since pulse-wave propagation velocity PWV changes in relation with change of blood pressure, the velocity PWV is used to monitor the blood pressure.

Since pulse-wave propagation velocity PWV is a velocity of propagation of a pulse wave in a blood vessel between two prescribed portions of a living subject, the velocity PWV is obtained by dividing a distance L (m) of propagation of the pulse wave between the two portions, by a time DT (sec) of propagation of the pulse wave between the two portions, according to the following Expression 1:

$$\text{(Expression 1)} \qquad PWV = L/DT$$

Since propagation distance L is present in the body of the subject, the distance L cannot be directly measured. Hence, propagation distance L is calculated based on a stature H of the subject, according to the following Expression 2 representing a relationship between propagation distance L and stature H that is experimentally determined, in advance:

$$\text{(Expression 2)} \qquad L = aH + b$$

where a, b are constants that are experimentally determined.

Meanwhile, since blood vessels lengthen and wind with age, propagation distance L increases with age, even if a distance on body surface between two portions may not change. In addition, contrary to the blood vessels, stature H decreases with age. However, the above-indicated relationship is used to calculate a propagation distance L, irrespective of an age of the subject. Therefore, the accuracy of the thus calculated propagation distance L is not sufficiently high, and accordingly the pulse-wave propagation velocity PWB determined based on the propagation distance L is not so high, either.

SUMMARY OF THE INVENTION

It is therefore an object of the present invention to provide a pulse-wave-propagation-velocity measuring apparatus which can determine an accurate pulse-wave propagation velocity.

The above object has been achieved by the present invention. According to the present invention, there is provided an apparatus for measuring a velocity of propagation of a pulse wave between two portions of a living subject, based on a distance, and a time, of propagation of the pulse wave between the two portions, the apparatus comprising a propagation-distance determining means for determining the distance of propagation of the pulse wave, based on stature-related information that is related to a stature of the subject and age-related information that is related to an age of the subject, according to a predetermined relationship between (A) propagation distance and (B) (b1) stature-related information and (b) age-related information; and a propagation-velocity determining means for determining the velocity of propagation of the pulse wave based on at least the distance of propagation determined by the propagation-distance determining means.

According to this invention, the propagation-distance determining means determines the distance of propagation of the pulse wave, based on both the stature-related information and the age-related information, that is, based on the age-related information in addition to the stature-related information. Therefore, the present apparatus can determine an accurate propagation distance and accordingly an accurate propagation velocity.

BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:

Fig. 1 is a diagrammatic view for explaining a construction of a superior-and-inferior-limb blood-pressure index measuring apparatus which has the function of measuring a pulse-wave propagation velocity and to which the present invention is applied;

Fig. 2 is a graph showing respective examples of a phonocardiogram detected by a microphone of the apparatus of Fig. 1, and a carotid pulse wave detected by a carotid-pulse-wave sensor of the same apparatus;

Fig. 3 is a diagrammatic view for explaining essential control functions of a control device of the apparatus of Fig. 1;

Fig. 4 is a graph showing a map which models a relationship between (A) corrected pulse-wave propagation velocity and (B) (b1) pulse-wave propagation velocity and (b2) diastolic blood pressure;

Fig. 5 is a flow chart representing a control program according to which the control device of the apparatus of Fig. 1 is operated; and

Fig. 6 is a graph showing a two-dimensional graph which is defined by a first axis indicative of superior-and-inferior-limb blood-pressure index and a second axis indicative of corrected pulse-wave propagation velocity and which is displayed by a display device of the apparatus of Fig. 1.

DETAILED DESCRIPTION OF PREFERRED EMBODIMENTS

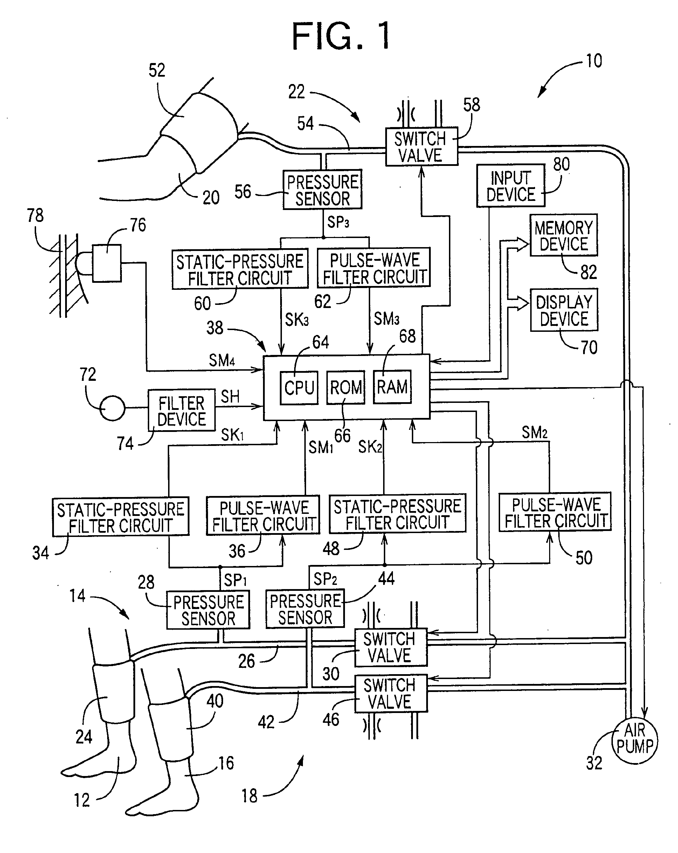

Hereinafter, there will be described an embodiment of the present invention in detail by reference to the drawings. Fig. 1 is a diagrammatic view for explaining a construction of a superior-and-inferior-limb blood-pressure (BP) index measuring apparatus 10 which has the function of measuring a pulse-wave propagation velocity and to which the present invention is applied. The present apparatus 10 carries out measurements on a patient as a living subject who takes a face-down, a lateral, or a face-up position so that an upper arm and ankles of the patient are substantially level with one another.

In Fig. 1, the superior-and-inferior-limb BP index measuring apparatus 10 includes a right-inferior-limb BP measuring device 14 which measures a BP value of a right ankle 12 of the patient, a left-inferior-limb BP measuring device 18 which measures a BP value of a left ankle 16 of the patient, and a superior-limb BP measuring device 22 which measures a BP value of an upper arm 20 of the patient.

The right-inferior-limb first BP measuring device 14 includes an inflatable cuff 24 which includes a belt-like cloth bag and a rubber bag accommodated in the cloth bag and which is adapted to be wound around the right ankle 12 of the patient; a piping 26; and a pressure sensor 28, a switch valve 30, and an air pump 32 which are connected to the cuff 24 via the piping 26. The switch valve 30 is selectively placed in one of three positions, that is, a pressure-supply position in which the switch valve 30 allows a pressurized air to be supplied from the air pump 32 to the cuff 24; a slow-deflation position in which the switch valve 30 allows the pressurized air to be slowly discharged from the cuff 24; and a quick-deflation position in which the switch valve 30 allows the pressurized air to be quickly discharged from the cuff 24.

The pressure sensor 28 detects an air pressure in the cuff 24, and supplies a pressure signal, SP1, representing the detected air pressure, to a static-pressure filter circuit 34 and a pulse-wave filter circuit 36. The static-pressure filter circuit 34 includes a low-pass filter which extracts, from the pressure signal SP1, a cuff pressure signal, SK1, representing a cuff pressure, PC1, as a static component of the detected air pressure. The filter circuit 34 supplies the cuff-pressure signal SK1 to a control device 38 via an analog-to-digital (A/D) converter, not shown.

The pulse-wave filter circuit 36 includes a band-pass filter which extracts, from the pressure signal SP1, a pulse-wave signal, SM1, representing a pulse wave as an oscillatory component of the detected air pressure that has prescribed frequencies. The filter circuit 36 supplies the pulse-wave signal SM1 to the control device 38 via an A/D converter, not shown.

The left-inferior-limb BP measuring device 18 includes an inflatable cuff 40, a piping 42, a pressure sensor 44, and a switch valve 46 which have respective constructions identical with those of the counterparts 24, 26, 28, 30 of the right-inferior-limb BP measuring device 14. The switch valve 46 is connected to the air pump 32. The pressure sensor 44 detects an air pressure in the cuff 40, and supplies a pressure signal, SP2, representing the detected air pressure, to a static-pressure filter circuit 48 and a pulse-wave filter circuit 50 which have respective constructions identical with those of the counterparts 34, 36 of the right-inferior-limb BP measuring device 14. The static-pressure filter circuit 48 extracts, from the pressure signal SP2, a cuff-pressure signal, SK2, representing a cuff pressure, PC2, as a static component of the detected air pressure, and supplies the cuff-pressure signal SK2 to the control device 38 via an A/D converter, not shown. The pulse-wave filter circuit 50 extracts, from the pressure signal SP2, a pulse-wave signal, SM2, representing a pulse wave as an oscillatory component of the detected air pressure that has prescribed frequencies, and supplies the pulse-wave signal SM2 to the control device 38 via an A/D converter, not shown.

The superior-limb BP measuring device 22 includes an inflatable cuff 52 which has a construction identical with the cuff 24 or 40 and which is adapted to be wound around the upper arm 20 (e.g., right upper arm) of the patient; and a piping 54, a pressure sensor 56, and a switch valve 58 which have respective constructions identical with those of the counterparts 26, 28, 30 of the right-inferior-limb BP measuring device 14. The switch valve 58 is connected to the air pump 32. The pressure sensor 56 detects an air pressure in the cuff 52, and supplies a pressure signal, SP3, representing the detected air pressure, to a static-pressure filter circuit 60 and a pulse-wave filter circuit 62 which have respective constructions identical with those of the counterparts 34, 36 of the right-inferior-limb BP measuring device 14. The static-pressure filter circuit 60 extracts, from the pressure signal SP3, a cuff-pressure signal, SK3, representing a cuff pressure, PC3, as a static component of the detected air pressure, and supplies the cuff-pressure signal SK3 to the control device 38 via an A/D converter, not shown. The pulse-wave filter circuit 62 extracts, from the pressure signal SP3, a pulse-wave signal, SM3, representing a pulse wave as an oscillatory component of the detected air pressure that has prescribed frequencies, and supplies the pulse-wave signal SM3 to the control device 38 via an A/D converter, not shown.

The control device 38 is essentially provided by a microcomputer including a central processing unit (CPU) 64, a read only memory (ROM) 66, a random access memory (RAM) 68, and an input-and-output (I/O) port, not shown, and processes input signals according to the control programs pre-stored in the ROM 66, while utilizing the temporary-storage function of the RAM 68. The control device 38 outputs, from the I/O port, drive signals to the air pump 32 and the three switch valves 30, 46, 58 to control the respective operations thereof, and additionally outputs display signals to a display device 70 to control what is displayed thereby.

A microphone 72 is attached, with an adhesive tape, not shown, to the skin of central portion of the chest of the patient, more specifically described, a prescribed heart-sound-detect position right above the apex cordis, the left end of the fourth intercostal sternum, the left end of the second intercostal sternum, the right end of the second intercostal sternum, or the right end of the fourth intercostal sternum. The microphone 72 detects heart sounds which are transmitted from the heart to the skin of the prescribed heart-sound-detect position. The heart sounds are produced when the heart starts outputting blood to the aorta, and when the heart ends outputting blood to the aorta. Thus, the heart sounds provide a pulse wave which is produced from the most upstream portion of the aorta. The microphone 72 functions as a first pulse-wave detecting device.

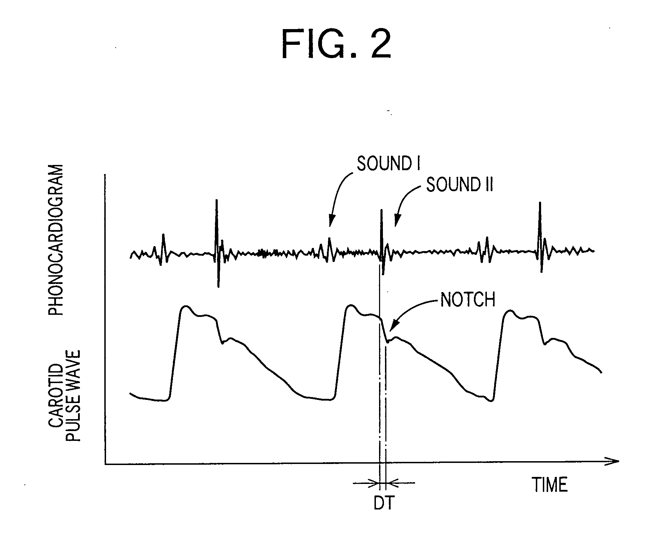

The microphone 72 includes a piezoelectric element, not shown, which converts the sounds detected thereby into an electric signal, i.e., a heart-sound signal, SH, and outputs the heart-sound signal SH, which subsequently is amplified by a preamplifier, not shown, and is supplied to a filter device 74. Then, the signal SH is supplied to the control device 38 via a main amplifier and an A/D converter, both not shown. The filter device 74 includes four sorts of filters, not shown, which can be so selected and used that the low-pitched-sound components of the heart-sound signal SH are attenuated and the high-pitched-sound components of the same SH are exaggerated and accordingly the heart sounds can be easily heard by the auditory sense of a human being. An upper half portion of Fig. 2 shows an example of a phonocardiogram detected by the microphone 72. The phonocardiogram includes a first sound I corresponding to the closing of the mitral valve and the opening of the aortic valve, and a second sound II corresponding to the closing of the aortic valve.

A carotid-pulse-wave sensor 76 functions as a second pulse-wave detecting device which is worn on a portion of the patient that is located on a downstream side of the microphone 72 as the first pulse-wave detecting device, as seen in the direction of flowing of blood in the body of the patient, and which detects a pulse wave propagating through an artery running in that portion of the patient. The carotid-pulse-wave sensor 76 includes a contact member, and a vibration sensor, not shown, which detects vibration of the contact member. The carotid-pulse-wave sensor 76 is attached to the neck of the patient such that the contact member of the sensor 76 is held in pressed contact with the skin right above a carotid artery 78 and detects a carotid-artery pulse wave produced from the carotid artery 78. The carotid-pulse-wave sensor 76 supplies a carotid-pulse-wave signal, SM4, representing the detected carotid-artery pulse wave, to the control device 38 via an A/D converter, not shown. A lower half portion of Fig. 2 shows an example of the carotid-artery pulse wave detected by the carotid-pulse-wave sensor 76. Since the carotid artery 78 has a considerably great diameter and is directly connected to the aorta, the waveform of the carotid-artery pulse wave is substantially identical with that of an aortic pulse wave produced from the aorta.

An input device 80 includes a keyboard, not shown, which is operated by an operator such as a doctor or a nurse to input stature-related information HI that is related to a stature H of the patient, and age-related information AI that is related to an age of the patient. The input device 80 outputs signals representing the stature-related information HI and the age-related information AI, to the control device 38. The stature-related information HI may be not only an actual stature or height H of the patient, but also a length of an arm of the patient or a height of the patient sitting down. The age-related information AI may be not only an actual age A of the patient but also a physiological age of the patient. The physiological age may be determined according to the method disclosed in Japanese Patent Document No. 3-15439 or its corresponding U.S. Patent No. 5,000,188. According to the disclosed method, all people are grouped into four groups, i.e., an infant-age group, a young-age group, a middle-age group, and an old-age group, and a memory device 82 stores respective reference pulse-wave patterns corresponding to the four age groups. One of the four reference pulse-wave patterns that best approximates an actual pattern of a pulse wave detected from the patient, is determined or selected, and the physiological age of the patient is determined as one of the four age groups that corresponds to the thus selected reference pulse-wave pattern. The memory device 82 may be provided by any one of well-known memory devices, such as a magnetic disk, a magnetic tape, a volatile semiconductor memory, or a non-volatile semiconductor memory. In addition, the memory device 82 stores, in respective prescribed memory areas thereof, an super-and-inferior-limb BP index (i.e., an ankle/arm blood pressure index; hereinafter, referred to as the "ABI") and pulse-wave-propagation-velocity-related information.

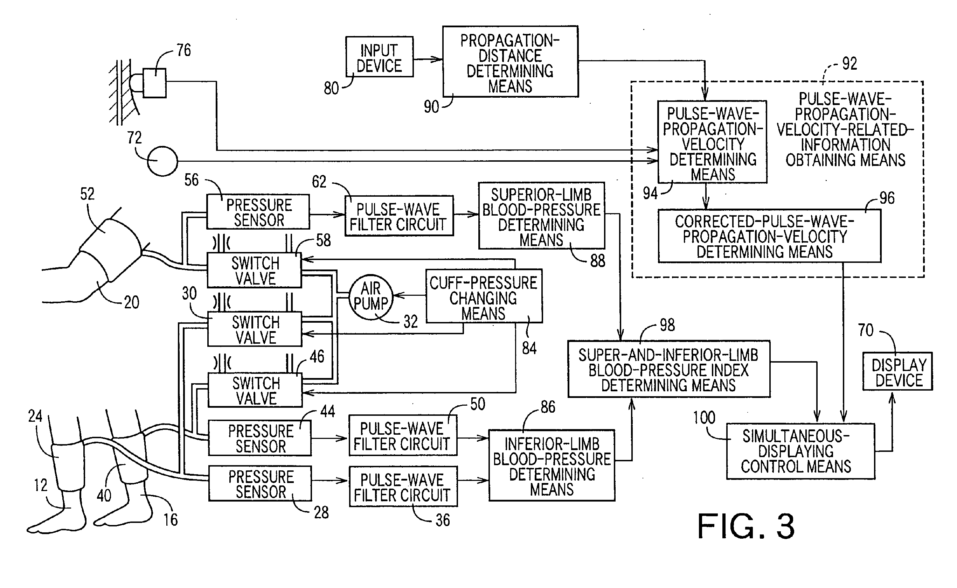

Fig. 3 is a diagrammatic view for explaining essential control functions of the control device 38. A cuff-pressure changing means 84 controls the air pump 32 and the three switch valves 30, 46, 58 each connected to the pump 32, such that the respective pressures of the three cuffs 24, 40, 52 are quickly increased up to predetermined target pressure values, $P_{CM}$, (e.g., 240 mmHg for the cuffs 24, 40 respectively wound around the ankles 12, 16, and 180 mmHg for the cuff 52 wound around the upper arm 20) and then are slowly decreased at a rate of about 3 mmHg/sec.

An inferior-limb-BP determining means 86 determines right-inferior-limb BP values, LBP(R), that is, BP values of the right ankle 12, according to well-known oscillometric method, based on the change of respective amplitudes of heartbeat-synchronous pulses of the pulse-wave signal SM1 detected one by one during the slow decreasing of the pressure of the cuff 24 wound around the right ankle 12 under the control of the cuff-pressure changing means 84. In addition, the inferior-limb-BP determining means 86 determines left-inferior-limb BP values, LBP(L), that is, BP values of the left ankle 16, according to the oscillometric method, based on the change of respective amplitudes of heartbeat-synchronous pulses of the pulse-wave signal SM2 detected one by one during the slow decreasing of the pressure of the cuff 40 wound around the left ankle 16 under the control of the cuff-pressure changing means 84. The right-inferior-limb BP values LBP(R) include a systolic BP value $LBP(R)_{SYS}$ and a diastolic BP value $LBP(L)_{DIA}$, and the left-inferior-limb BP values LBP(L) include a systolic BP value $LBP(L)_{SYS}$ and a diastolic BP value $LBP(L)_{DIA}$. Hereinafter, when it is not needed to distinguish the right-inferior-limb BP values LBP(R) and the left-inferior-limb BP values LBP(L) from each other, those BP values as a whole will be referred to as the inferior-limb BP values LBP.

A superior-limb-BP determining means 88 determines superior-limb BP values, ABP, (e.g., systolic BP value $ABP_{SYS}$ and diastolic BP value $ABP_{DIA}$), that is, BP values of the upper arm 20, according to the well-known oscillometric method, based on the change of respective amplitudes of heartbeat-synchronous pulses of the pulse-wave signal SM3 detected one by one during the slow decreasing of the pressure of the cuff 52 wound around the upper arm 20 under the control of the cuff-pressure changing means 84.

A propagation-distance determining means 90 determines, based on the stature-related information HI and the age-related information AI input through the input device 80, a propagation distance L (m), i.e., a length of an artery as measured from the aortic valve to a position where the carotid-pulse-wave sensor 76 is pressed against the carotid artery 78, according to a prescribed relationship defined by the following Expression 3:

$$\text{(Expression 3)} \qquad L = \alpha \, HI \times \beta \, AI + \gamma$$

where $\alpha$, $\beta$, $\gamma$ are constants.

The constants $\alpha$, $\beta$, $\gamma$ are determined, in advance, based on, e.g., lengths of arteries actually measured by anatomy, or statistic analysis of a number of combinations of stature-related information HI and age-related information AI.

In the case where the age-related information AI is expressed by not a value such as an actual age of the patient, but, e.g., a selected one of the above-described age groups, those age groups are expressed by respective predetermined values C, and the propagation distance L is determined according to the following Expression 4:

$$\text{(Expression 4)} \qquad L = \varepsilon \, HI \times C + \delta$$

where $\varepsilon$, $\delta$ are constants.

The constants $\varepsilon$, $\delta$ of Expression 4 are determined, in advance, in the same manner as that in which the constants $\alpha$, $\beta$, $\gamma$ of Expression 3 are determined.

A pulse-wave-propagation-velocity determining means 94 as part of a pulse-wave-propagation-velocity-relating-information obtaining means 92 determines a pulse-wave propagation time DT, i.e., a time needed for a pulse wave to propagate from the position where the first pulse-wave detecting device detects the pulse wave, to the position where the second pulse-wave detecting device detects the pulse wave. In the present embodiment, the pulse-wave-propagation-velocity determining means 94 determines, as a pulse-wave propagation time DT, a time needed for a pulse wave to propagate from the aortic valve to the position where the carotid-pulse-wave sensor 76 is pressed against the carotid artery 78, based on the heart sounds detected by the microphone 72 and the carotid pulse wave detected by the carotid-pulse-wave sensor 76. In addition, the determining means 94 determines, based on the propagation distance L determined by the propagation-distance determining means 90 and the thus determined pulse-wave propagation time DT, a pulse-wave propagation velocity PWV (m/sec), according to the previously-indicated, Expression 1.

For example, the pulse-wave propagation time DT may be determined, as illustrated in Fig. 2, as a time difference between a time point when the microphone 72 detects a starting point of a second heart sound II (this point corresponds to a notch of the aortic pulse wave where its amplitude ends quick decreasing and starts increasing), and a time point when the carotid-pulse-wave sensor 76 detects a notch of the carotid pulse wave.

A corrected-pulse-wave-propagation-velocity determining means 96 as another part of the pulse-wave-propagation-velocity-relating-information obtaining means 92 corrects the pulse-wave propagation velocity PWV determined by the pulse-wave-propagation-velocity determining means 94, into a corrected pulse-wave propagation velocity PWVc corresponding to a prescribed BP value (e.g., 80 mmHg). This corrected pulse-wave propagation velocity PWVc is generally known as an index which can be used to evaluate a degree of arteriosclerosis of a whole body of a patient. The corrected pulse-wave propagation velocity PWVc may be determined by any of various known methods; for example, a first method (1) in which the pulse-wave propagation velocity PWV is corrected based on a blood-pressure value BP determined by a BP determining means, such as the inferior-limb-BP determining means 86 or the superior-limb-BP determining means 88, that determines a BP value of a prescribed portion of a living subject, or a second method (2) in which the corrected pulse-wave propagation velocity PWVc is determined, according to a prescribed relationship between (A) corrected pulse-wave propagation velocity PWVc and (B) (b1) pulse-wave propagation velocity PWV and (b2) blood-pressure-related-information that changes in relation with change of blood pressure, based on a pulse-wave propagation velocity PWV and a piece of blood-pressure-related information that are actually obtained from a living subject. The blood-pressure-related information may be an ejection period ET during which blood is ejected from the left ventricle of the heart of a living subject; a pre-ejection period PEP from the starting of contraction of left-ventricular muscle of the heart of a living subject to the starting of ejection of blood from the left ventricle; or a heart rate HR.

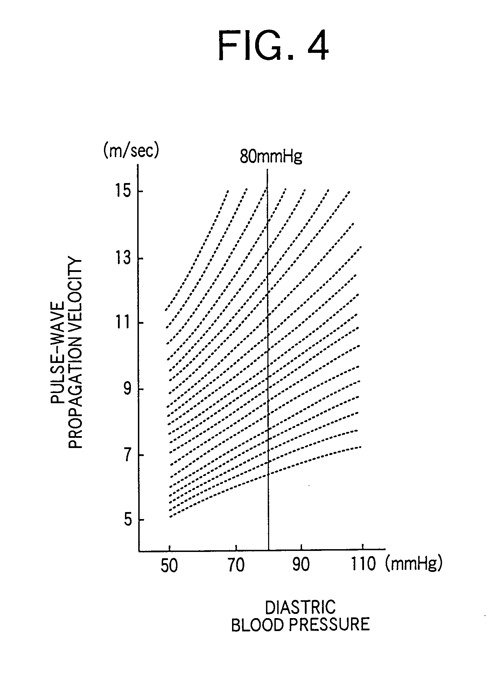

In the case where the above-indicated first method (1) is employed to determine the corrected pulse-wave propagation velocity PWVc, the ROM 66, for example, is used to store a map, shown in Fig. 4, that models a prescribed relationship between (C) corrected pulse-wave propagation velocity PWVc and (D) (c1) pulse-wave propagation velocity PWV and (c2) diastolic blood pressure. The corrected-pulse-wave-propagation-velocity determining means 96 selects, from the map stored in the ROM 66, one of a plurality of curves (indicated at broken lines) that is the nearest to a point defined by the combination of the diastolic BP value $BP_{DIA}$ (the inferior-limb diastolic BP value $LBP_{DIA}$ or the superior-limb diastolic BP value $ABP_{DIA}$) actually determined by the above-indicated BP determining means and the pulse-wave propagation velocity PWV actually determined by the pulse-wave-propagation-velocity determining means 94. The determining means 96 determines, as a corrected pulse-wave propagation velocity PWVc, a value indicated by the selected curve at 80 mmHg.

A superior-and-inferior-limb BP index determining means 98 determines an index value ABI based on the inferior-limb BP value LBP determined by the inferior-limb-BP determining means 86, and the superior-limb BP value ABP determined by the superior-limb-BP determining means 88 that corresponds to the inferior-limb BP value LBP (for example, the superior-limb diastolic BP value $ABP_{DIA}$ corresponds to the inferior-limb diastolic BP value $LBP_{DIA}$, and the superior-limb systolic BP value $ABP_{SYS}$ corresponds to the inferior-limb systolic BP value $LBP_{SYS}$). The index value ABI may be determined by dividing the inferior-limb BP value LBP by the superior-limb BP value ABP, or dividing the superior-limb BP value ABP by the inferior-limb BP value LBP.

A simultaneous-displaying control means 100 controls the display device 70 to simultaneously display the pulse-wave-propagation-velocity-related information obtained by the pulse-wave-propagation-velocity-related information obtaining means 92 and the index value ABI determined by the superior-and-inferior-limb BP index determining means 98. For example, the display device 70 simultaneously displays the pulse-wave-propagation-velocity-related information (in digits) and the index value ABI (in digits), side by side. Alternatively, the display device 70 displays, in a two-dimensional screen thereof that is defined by a first axis indicative of superior-and-inferior-limb BP index and a second axis indicative of pulse-wave-propagation-velocity-related information, a mark or a symbol at a position corresponding to the actually determined index value ABI and the actually obtained pulse-wave-propagation-velocity-related information.

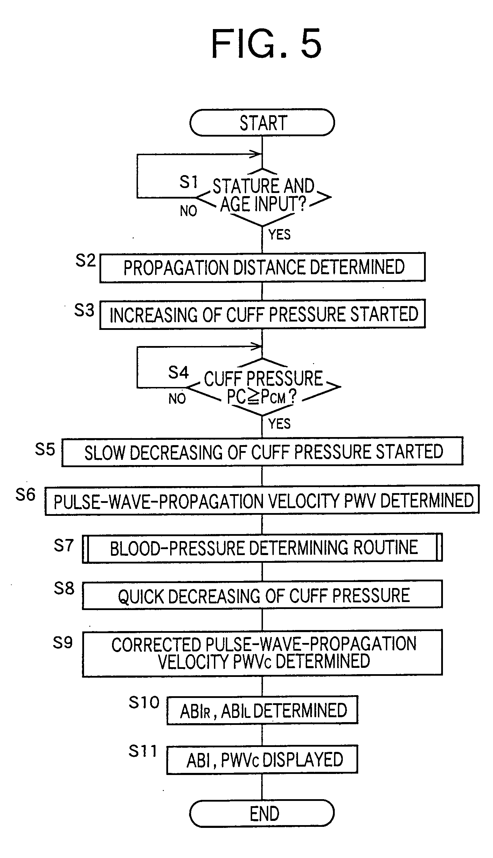

Fig. 5 is a flow chart representing a control program according to which the control device 38 is operated. In the flow chart of Fig. 5, first, the control device 38 carries out Step S1 (hereinafter, "Step" is omitted, if appropriate) to judge whether the control device has received the signals representing the stature H and the age A of the patient. So long as negative judgments are made at S1, S1 is repeated while the control device waits for receiving the signals. When a positive judgment is made at S1, the control goes to S2 corresponding to the propagation-distance determining means 90. At S2, the control device determines a propagation distance L based on the stature H and the age A received at S1, according to the above-explained, Expression 3. Since in this case the stature H is used as the stature-related information HI and the age A is used as the age-related information AI, the constants $\alpha$, $\beta$ of Expression 3 are positive values, and the propagation distance L increases as the stature H or the age A increases.

Then, the control goes to S3, S4, and S5 corresponding to the cuff-pressure changing means 84. At S3, the three switch valves 30, 46, 58 are switched to their pressure-supply positions and the air pump 32 is operated, so that the respective pressures of the three cuffs 24, 40, 52 are quickly increased. At S42, the control device judges whether the pressure PC of each of the three cuffs 24, 40, 52 has reached a prescribed target pressure value $P_{CM}$ (e.g., 240 mmHg for each of the cuffs 24, 40; and 180 mmHg for the cuff 52). If a negative judgment is made at S4, Steps S3 and S4 are repeated to continue increasing the pressures PC of the cuffs 24, 40, 52.

If a positive judgment is made at S4 for one of the cuffs 24, 40, 52, the control goes to S5 to switch a corresponding one of the switch valves 30, 46, 58 to its slow-deflation position, so that the pressure PC of the one cuff is slowly decreased at a prescribed low rate of about 3 mmHg/sec. When a positive judgment is made at S4 for the last one of the cuffs 24, 40, 52, the control device stops, at S5, the operation of the air pump 32 in addition to switching the corresponding one of the switch valves 30, 46, 58 to its slow-deflation position.

Then, at S6 corresponding to the pulse-wave-propagation-velocity-relating-information obtaining means 94, the control device determines, as illustrated in Fig. 2, a pulse-wave propagation time DT as a time difference between a time when a starting point of a second heart sound II occurs to the heart-sound signal SH continuously supplied from the microphone 72, and a time when a notch occurs to the carotid-pulse-wave signal $SM_4$ continuously supplied the carotid-pulse-wave sensor 76. In addition, the control device determines a pulse-wave propagation velocity PWV based on the thus determined pulse-wave propagation time DT and the propagation distance L determined at S2, according to the above-indicated Expression 1.

Then, the control goes to S7, i.e., the blood-pressure determining routine corresponding to the inferior-limb-BP determining means 86 and the superior-limb-BP determining means 88. More specifically described, the control device 38 determines an amplitude of each of successive heartbeat-synchronous pulses of the cuff pulse wave represented by the pulse-wave signal SM1 continuously supplied from the pulse-wave filter circuit 36, and determines a right-inferior-limb systolic BP value $LBP(R)_{SYS}$, etc. based on the time-wise change of the thus determined amplitudes according to well-known oscillometric BP-determining algorithm. Similarly, the control device 38 determines an amplitude of each of successive heartbeat-synchronous pulses of the cuff pulse wave represented by the pulse-wave signal SM2 continuously supplied from the pulse-wave filter circuit 50, and determines a left-inferior-limb systolic BP value $LBP(L)_{SYS}$, etc. based on the time-wise change of the thus determined amplitudes according to the oscillometric BP-determining algorithm. In addition, the control device 38 determines an amplitude of each of successive heartbeat-synchronous pulses of the cuff pulse wave represented by the pulse-wave signal SM3 continuously supplied from the pulse-wave filter circuit 62, and determines a superior-limb systolic BP value $ABP_{SYS}$, etc. based on the time-wise change of the thus determined amplitudes according to the oscillometric BP-determining algorithm.

When all the blood-pressure values BP are determined according to the blood-pressure determining routine at S6, the control goes to S8 corresponding to the cuff-pressure changing means 84. At S8, the control device switches the three switch valves 30, 46, 58 to their quick-deflation positions, so that the respective pressures of the three cuffs 24, 40, 52 are quickly lowered.

Then, the control goes to S9 corresponding to the corrected-pulse-wave-propagation-velocity determining means 96. At S9, the control device selects one of a plurality of curves of the map, shown in Fig. 4 and stored in the ROM 66, that is the nearest to a point defined by the pulse-wave propagation velocity PWV determined at S6 and the superior-limb diastolic BP value $ABP_{DIA}$ determined at S7, and determines, as a corrected pulse-wave propagation velocity PWVc, a value indicated by the selected curve at 80 mmHg.

Then, the control goes to S10 corresponding to the superior-and-inferior-limb BP index determining means 98. At S10, the control device calculates a right-inferior-limb-side index value $ABI_R$ by dividing, by the superior-inferior-limb systolic BP value $ABP_{SYS}$ determined at S7, the right-inferior-limb systolic blood pressure $LPB(R)_{SYS}$ determined at S7, and calculates a left-inferior-limb-side index value $ABI_L$ by dividing, by the superior-limb systolic BP value $ABP_{SYS}$ determined at S7, the left-inferior-limb systolic BP value $LBP(L)_{SYS}$ determined at Step S7.

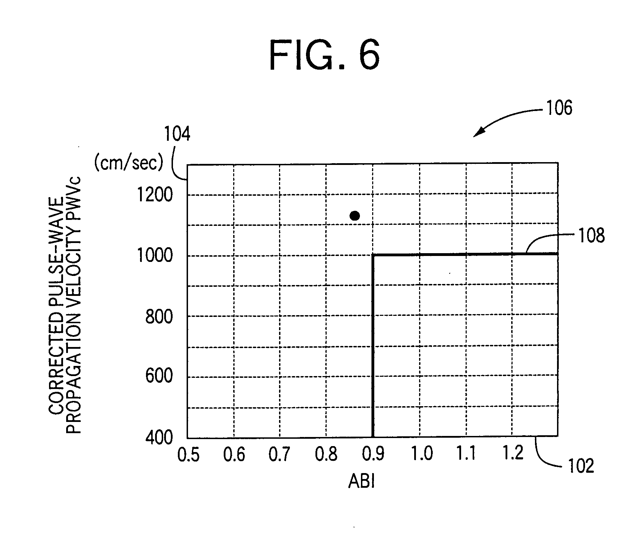

Then, at S11 corresponding to the simultaneous-displaying control means 100, the control device controls the display device 70 to display, in a two-dimensional graph 106 that is defined by an ABI axis 102 and a corrected-pulse-wave-propagation-velocity axis 104, as shown in Fig. 6, and is displayed on an image screen of the display device 70, a symbol "●" at a position defined by the corrected pulse-wave propagation velocity PWVc determined at S9 and the lower one of the right-inferior-limb-side index value $ABI_R$ and the left-inferior-limb-side index value $ABI_L$ each determined at S10. The two-dimensional graph 106 shows a reference line 108 representing a boundary between a normal range (not smaller than 0.9), and an abnormal range, of the index value ABI that are experimentally determined in advance, and a boundary between a normal range (not greater than 1,000 (cm/sec)), and an abnormal range, of the pulse-wave propagation velocity PWVc that are also experimentally determined in advance, so that the operator may easily see the diagnosis made by the apparatus 10, i.e., whether the symbol "●" falls in each of the two normal ranges or at least one of the two abnormal ranges.

Since the symbol "●" is indicated at the position defined by the index value ABI and the corrected pulse-wave propagation velocity PWVc in the two-dimensional graph 106, the operator can simultaneously judge whether the index value ABI is abnormal and whether the corrected pulse-wave propagation velocity PWVc is abnormal. Therefore, the operator can make the following judgments: In the case where the index value ABI is abnormal and the corrected pulse-wave propagation velocity PWVc is normal, it can be judged that the patient is highly suspected to have arteriostenosis in his or her inferior limb or limbs. In addition, In the case where the corrected pulse-wave propagation velocity PWVc is abnormal, it can be judged that the patient is suspected to suffer advanced systemic arteriosclerosis and that even if the index value ABI may be normal, he or she is suspected to have arteriostenosis in his or her inferior limb or limbs.

As described above, in the illustrated embodiment in which the flow chart of Fig. 5 is employed, at S2 (the propagation-distance determining means 90), the control device 38 calculates the propagation distance L based on the stature H and the age A input through the input device 80, according to Expression 3. That is, since the propagation distance L is determined by taking not only the stature H but also the age A into consideration, the thus determined propagation distance L enjoys a high accuracy.

In addition, since the pulse-wave propagation velocity PWV is determined based on the accurate propagation distance L, the thus determined pulse-wave propagation velocity PWV enjoys a high accuracy; and since the corrected pulse-wave propagation velocity PWVc is determined based on the accurate pulse-wave propagation velocity PWV, the corrected pulse-wave propagation velocity PWVc also enjoys a high accuracy. This contributes to making an accurate diagnosis based on the corrected pulse-wave propagation velocity PWVc.

While the present invention has been described in its preferred embodiment by reference to the drawings, it is to be understood that the invention may otherwise be embodied.

For example, in the illustrated superior-and-inferior-limb BP index measuring apparatus 10, the microphone 72 which detects the heart sounds is employed as the first pulse-wave detecting device to be used to measure the pulse-wave propagation velocity PWV. However, the first pulse-wave detecting device may be provided by an electrocardiograph which includes a plurality of electrodes adapted to be worn on a living subject, detects, through those electrodes, an action potential of cardiac muscle of the subject, and continuously outputs an electrocardiogram representing the detected action potential of cardiac muscle. In the latter case, however, the pulse-wave propagation time DT involves a pre-ejection period PEP from the starting of contraction of cardiac muscle of the left ventricle of the subject to the starting of ejection of blood from the left ventricle. To solve this problem, in place of Expression 1, the following Expression 5 is employed to determine the pulse-wave propagation velocity PWV. In Expression 5, PEP is a constant value which is experimentally determined in advance.

$$\text{(Expression 5)} \qquad PWV = L/(DT - PEP)$$

In addition, in the illustrated superior-and- inferior-limb BP index measuring apparatus 10, the input device 80 is used to input the stature H and the age A of the patient. However, the memory device 82 may be adapted to store, in advance, stature-related information HI and age-related information of each of individual patients. In the latter case, the input device 80 may be used to input, for each patient to be measured, information (e.g., an identification number of the each patient) which enables the apparatus 10 to identify and read the stature-related information HI and the age-related information of the each patient, from all the sets of stature-related information HI and age-related information stored in the memory device 82.

It is to be understood that the present invention may be embodied with other changes, improvements and modifications that may occur to a person skilled in the art without departing from the spirit and scope of the invention defined in the appended claims.

## Claims

1. An apparatus (10) for measuring a velocity of propagation of a pulse wave between two portions of a living subject, based on a distance, and a time, of propagation of the pulse wave between the two portions, the apparatus comprising:

   a propagation-distance determining means (90) for determining the distance of propagation of the pulse wave, based on stature-related information that is related to a stature of the subject and age-related information that is related to an age of the subject, according to a predetermined relationship between (A) propagation distance and (B) (b1) stature-related information and (b) age-related information; and
   a propagation-velocity determining means (94) for determining the velocity of propagation of the pulse wave based on at least the distance of propagation determined by the propagation-distance determining means.

2. An apparatus according to claim 1, further comprising a propagation-time measuring device (72, 76, 94) which measures the time of propagation of the pulse wave, wherein the propagation-velocity determining means (94) determines the velocity of propagation of the pulse wave based on the distance of propagation determined by the propagation-distance determining means and the time of propagation measured by the propagation-time measuring device.

3. An apparatus according to claim 2, wherein the propagation-time measuring device (72, 76, 94) comprises:

   a first pulse-wave detecting device (72) which detects a first pulse wave from one of the two portions of the subject;
   a second pulse-wave detecting device (76) which detects a second pulse wave from the other of the two portions of the subject; and means (94) for determining, as the time of propagation of the pulse wave, a time difference between a time of occurrence of a periodic point to the first pulse wave detected by the first pulse-wave detecting device and a time of occurrence of a corresponding periodic point to the second pulse wave detected by the second pulse-wave detecting device.

4. An apparatus according to any one of claims 1 to 3, further comprising an input device (80) which is operable for inputting at least one of the stature-related information and the age-related information.

5. An apparatus according to any one of claims 1 to 4, further comprising a memory device (66) which stores data representing the predetermined relationship between (A) propagation distance and (B) (b1) stature-related information and (b) age-related information.

6. An apparatus according to any one of claims 1 to 5, further comprising a corrected-propagation-velocity determining device (86, 88, 96) which corrects the velocity of propagation of the pulse wave into a corrected propagation velocity.

7. An apparatus according to claim 6, wherein the corrected-propagation-velocity determining device (24, 40, 52, 86, 88, 96) comprises:

a blood-pressure-related-information obtaining device (24, 40, 52, 86, 88) which obtains blood-pressure-related-information that changes in relation with a blood pressure of the subject; and

means (96) for correcting the velocity of propagation of the pulse wave into the corrected propagation velocity, based on the blood-pressure-related-information obtained by the blood-pressure-related-information obtaining device.

8. An apparatus according to claim 7, wherein the blood-pressure-related-information obtaining device (24, 40, 52, 86, 88) comprises a blood-pressure measuring device (24, 40, 52) which measures, as the blood-pressure-related-information, a blood pressure of the subject.

9. An apparatus according to any one of claims 1 to 8, further comprising a superior-and-inferior-limb blood-pressure index measuring device (24, 40, 52, 86, 88, 98) which measures a superior-and-inferior-limb blood-pressure index of the subject.

10. An apparatus according to claim 9, wherein the superior-and-inferior-limb blood-pressure index measuring device (24, 40, 52, 86, 88, 98) comprises:

a superior-limb blood-pressure measuring device (52, 88) which measures a superior-limb blood-pressure of a superior limb of the subject; an inferior-limb blood-pressure measuring device (24, 40, 86) which measures an inferior-limb blood-pressure of an inferior limb of the subject; and

an index determining means (98) for determining the superior-and-inferior-limb blood-pressure index of the subject, based on the superior-limb blood-pressure measured by the superior-limb blood-pressure measuring device and the inferior-limb blood-pressure measured by the inferior-limb blood-pressure measuring device.

11. An apparatus according to claim 9 or claim 10, further comprising:

a display device (70); and
a simultaneous-displaying control means (100) for controlling the display device to simultaneously display the velocity of propagation of the pulse wave determined by the propagation-velocity determining means (94), and the superior-and-inferior-limb blood-pressure index measured by the superior-and-inferior-limb blood-pressure index measuring device (24, 40, 52, 86, 88, 98).

12. An apparatus according to any one of claims 6 to 11, further comprising a display device (70) which displays the corrected propagation velocity determined by the corrected-propagation-velocity determining means (96).

13. An apparatus according to claim 12, further comprising:

a superior-and-inferior-limb blood-pressure index measuring device (24, 40, 52, 86, 88, 98) which measures a superior-and-inferior-limb blood-pressure index of the subject;
a display device (70); and
a simultaneous-displaying control means (100) for controlling the display device to simultaneously display the corrected propagation velocity determined by the corrected-propagation-velocity determining means (96), and the superior-and-inferior-limb blood-pressure index measured by the superior-and-inferior-limb blood-pressure index measuring device.

# FIG. 1

# FIG. 2

FIG. 3

# FIG. 4

# FIG. 5

START

S1 STATURE AND AGE INPUT?

NO

YES

S2 PROPAGATION DISTANCE DETERMINED

S3 INCREASING OF CUFF PRESSURE STARTED

S4 CUFF PRESSURE PC≧PCM?

NO

YES

S5 SLOW DECREASING OF CUFF PRESSURE STARTED

S6 PULSE-WAVE-PROPAGATION VELOCITY PWV DETERMINED

S7 BLOOD-PRESSURE DETERMINING ROUTINE

S8 QUICK DECREASING OF CUFF PRESSURE

S9 CORRECTED PULSE-WAVE-PROPAGATION VELOCITY PWVc DETERMINED

S10 ABIR, ABIL DETERMINED

S11 ABI, PWVc DISPLAYED

END

# FIG. 6